(19) Europäisches Patentamt
European Patent Office
Office européen des brevets

(11) Numéro de publication : **0 046 113**
**B1**

(12) **FASCICULE DE BREVET EUROPÉEN**

(45) Date de publication du fascicule du brevet : **19.12.84**

(51) Int. Cl.$^3$ : **C 07 C103/52**

(21) Numéro de dépôt : **81401263.9**

(22) Date de dépôt : **05.08.81**

# (54) Nouveaux peptides et leur application en thérapeutique.

(30) Priorité : **08.08.80 FR 8017523**

(43) Date de publication de la demande :
**17.02.82 Bulletin 82/07**

(45) Mention de la délivrance du brevet :
**19.12.84 Bulletin 84/51**

(84) Etats contractants désignés :
**AT BE CH DE FR GB IT LI LU NL SE**

(56) Documents cités :
**DE-A- 2 741 393**
**FR-A- 2 338 925**
**FR-A- 2 347 336**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

(72) Inventeur : **Roques, Bernard**
**12 Rue Eugène Delacroix**
**F-94410 Saint Maurice (FR)**
Inventeur : **Lecomte, Jeanne Marie**
**329 Rue Lecourbe**
**F-75015 Paris (FR)**

(74) Mandataire : **Moncheny, Michel et al**
**c/o Cabinet Lavoix 2 Place d'Estienne d'Orves**
**F-75441 Paris Cedex 09 (FR)**

**EP 0 046 113 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne de nouveaux peptides ayant une activité importante sur les récepteurs opiacés δ, leur procédé de préparation et leur application en thérapeutique notamment comme analgésiques et psychotropes.

La présente invention concerne plus spécialement des penta et hexa peptides ayant une action importante sur les récepteurs opiacés δ.

On a déjà décrit des pentapeptides présentant une certaine analogie de structures dans le brevet FR 78 12 543. Toutefois, ces composés connus n'ont pratiquemment pas d'activité sur les récepteurs opiacés δ.

On sait qu'il existe au moins deux types de récepteurs opiacés (μ et δ) dans le cerveau (KOSTERLITZ et al. (1980), Brit. J. Pharmacol., *68*, 333-42, CHANG et al. (1979), J. Biol. Chem., *254*, 2610-18).

Ces deux types de récepteurs sont impliqués dans l'analgésie mais les récepteurs δ semblent également être impliqués dans le comportement, les états émotionnels, etc... (RICHTER et al. (1980) Life Sciences *26*, 337-42).

Les substances ayant une activité spécifique sur les récepteurs δ présentent normalement des effets d'accoutumance et de dépendance très inférieurs à ceux engendrés par les opiacés classiques (morphine, pethidine) ou par les dérivés d'enképhalines actuellement décrits (Frederickson et al. Science, *211*, 603-605, (1981).

Le brevet FR-A-2 338 925 décrit des hexapeptides présentant en position 2 de la chaîne peptidique, un groupe D. Ala, c'est-à-dire dont la chaîne latérale n'est pas hydroxylée. Ces composés par ailleurs sont spécifiques vis-à-vis des récepteurs μ.

Le brevet FR-A-2 347 336 décrit des peptides dont la fraction terminale est un groupe ester mais ne représente jamais un groupe amide. Ces peptides sont également spécifiques des récepteurs μ.

La présente invention vise donc à fournir des composés ayant une activité spécifique importante sur un seul type de récepteurs, à savoir les récepteurs δ.

La présente invention a pour objet des dérivés de peptides répondant à la formule générale

Tyr-A-Gly-B-C-D (I)

dans laquelle

A est un reste AzaGly, Aib, D. Ser, D. Thr, D. Cys, homo Sérine, β Phe Ser, β OH Leu, 4OH Ile, α, β, γ OHNor Val ou OH Val dont les groupes OH ou SH latéraux, lorsqu'ils existent, peuvent être libres ou protégés

i) par un radical alkyle simple ou ramifié ayant de 1 à 6 atomes de carbone,

ii) par un radical phényle non substitué ou substitué par un ou plusieurs atomes de fluor,

iii) par un radical benzyle non substitué ou substitué par un ou plusieurs atomes de fluor,

iv) par un radical acyle aliphatique ayant de 1 à 6 atomes de carbone ou par un radical acyle COX dans lesquels X est un radical phényle, benzyle ou benzyhydryle, éventuellement substitué par un ou plusieurs atomes de fluor.

B est un reste L. Phe, pF. L. Phe ou pentafluoro L. Phe,

C est un reste Leu, N. Leu ou Ile,

D représente un groupe

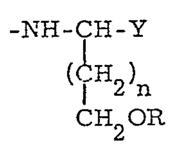

ou

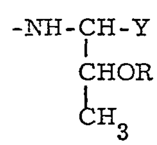

dans lequel

n = 0, 1 ou 2,

R est un atome d'hydrogène ou un radical tel que défini pour la protection du groupe OH du reste A,

Y est un atome d'hydrogène, un groupe hydroxy, carboxy, carbamoyle, un groupe $OR_1$, $COOR_1$ ou $CONHR_1$ dans lequel $R_1$ représente un radical tel que défini pour la protection du groupe OH du reste A, une chaîne phosphatidyléthanolamine ou une chaîne

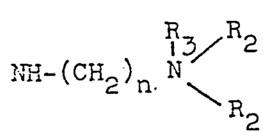

dans laquelle n est un nombre entier de 0 à 3, $R_2$ est un atome d'hydrogène ou un radical alkyle simple

ayant de 1 à 4 atomes de carbone et $R_3$ est un atome d'hydrogène ou d'oxygène ou un reste alkyle simple ayant de 1 à 4 atomes de carbone ainsi que leurs sels pharmaceutiquement acceptables.

Les composés de formule (I) peuvent être préparés par les procédés classiques de la synthèse peptidique. Ainsi, on peut les obtenir par condensation fragmentaire ou par couplage en série d'aminoacides convenablement choisis et protégés. La réaction de couplage s'effectue par les méthodes d'activation habituelles et les méthodes de déprotection classiquement utilisées en synthèse peptidique.

Des détails concernant ces techniques classiques peuvent être trouvés dans les références bibliographiques suivantes :

1. C. Garbay-Jaureguiberry, B.P. Roques, R. Oberlin, M. Anteunis et A.K. Lala B.B.R.C., *71*, 558-565, 1976.

2. C. Garbay-Jaureguiberry, B.P. Roques, R. Oberlin, M. Anteunis, S. Combrisson et J.Y. Lallemand Febs Letters, *76*, 93-98, 1977.

3. J.D. Bower et al., J. Chem. Soc. Perkin Trans I, 2488-92.

4. W. Woelther et al., Augen. Chem., Int. Ed. Engl., *15*, 297 (1976).

5. E. Pietrzik et al., Liebigs. Ann. Chem. p. 609 (1977).

A titre d'exemple général, les composés de la formule I peuvent être préparés par condensation d'un résidu M Tyr-OH dans lequel M représente un groupe de protection de la fonction amino (tertiobutyloxycarbonyle, benzyloxycarbonyle, fluorénylméthyloxycarbonyle) avec un résidu H-A-P où P est un groupe protecteur de la fonction acide (ester par exemple). La combinaison s'effectue par utilisation des agents d'activation classique et plus particulièrement du dicyclohexylcarbodiimide et de l'hydroxybenzotriazole.

L'élimination sélective du groupe protecteur N permet de continuer la synthèse par condensation successive jusqu'à l'obtention du peptide désiré.

Dans le cas de la synthèse par fragment les procédés utilisés sont semblables mais des ensembles de résidus sont alors couplés selon le schéma

M Tyr-A-OH + H-Gly-B-C-D-P.

L'invention est illustrée par les exemples suivants.

Dans ces exemples, les abréviations suivantes ont été utilisées.

Boc = tertio-butyloxycarbonyle
t.bu = tertiobutyle
DCC = dicyclohexylcarbodiimide
HOBT = hydroxybenzotriazole
Z = benzyloxycarbonyle
DCU = dicyclohexylurée
AcOET = acétate d'éthyle
THF = tétrahydrofuranne
$NEt_3$ = triéthylamine
TFA = acide trifluoroacétique

Les divers composés obtenus ont été caractérisés par résonance magnétique nucléaire (270-MHz) et leur pureté testée par chromatorgraphie en couche mince (CCM) dans les solvants suivants :

$C_1$ = $BuOH/ACOH/H_2O$, 4/1/1
$C_2$ = $CHCl_3/MeOH$, 7/3
$C_3$ = $CHCl_3/MeOH$ 9/1

ou par chromatographie liquide haute performance (HPLC).

Exemple 1

L. Tyr-D. Ser-Gly-Phe-Leu-Thr(XI)

On prépare ce composé en suivant le schéma suivant

(Voir tableau page suivante)

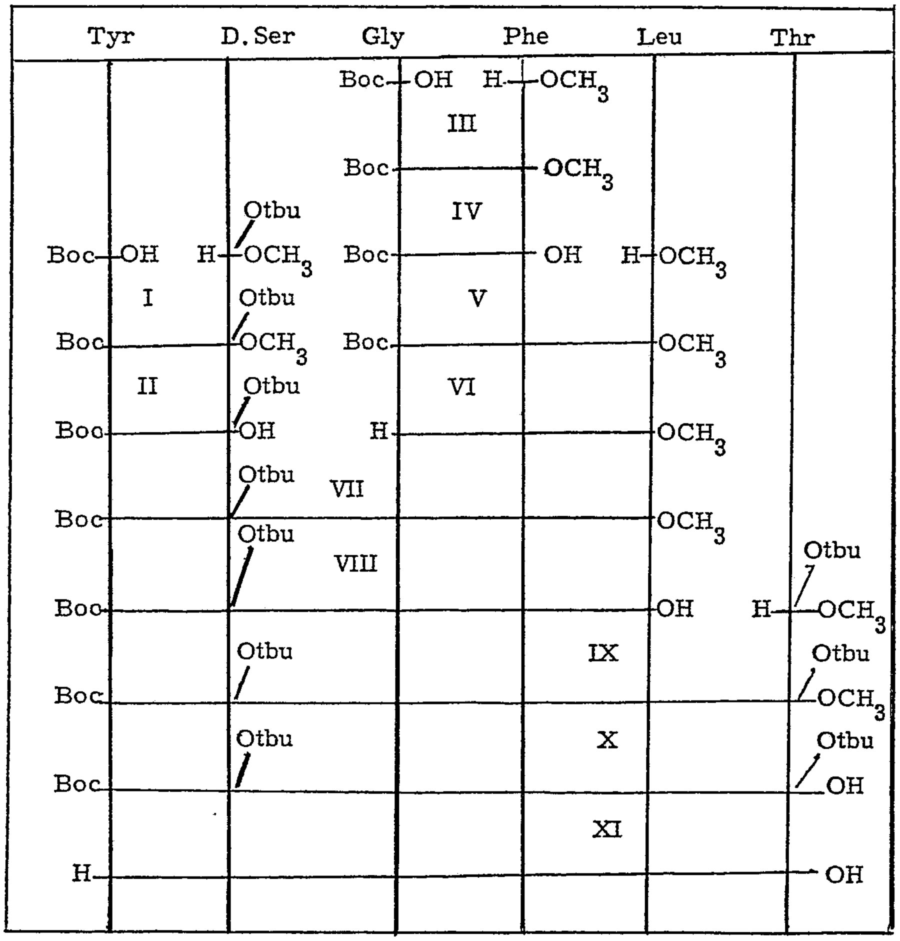

N-Boc-L. Tyrosyl-(Otbu) D. Sérine méthyl ester I

On place 3,37 g de Boc L. Tyr dans 20 ml de THF anhydre. On ajoute à 0 °C successivement 2,54 g de chlorhydrate de (Otbu) D. Sérine méthyl ester et 1,88 g de $NEt_3$ en solution dans 20 ml de $CHCl_3$ anhydre, puis 1,84 g de HOBT dans 10 ml de $CHCl_3$ anhydre et enfin 2,47 g de DCC en solution dans 20 ml de $CHCl_3$ sec. On agite 1 h à 0 °C puis 12 h à température ordinaire. On filtre la DCU, on évapore à sec sous vide. On reprend par 50 ml de AcOEt. On filtre la DCU insoluble. On lave avec 3 × 20 ml d'acide citrique, 1 × 20 ml de $H_2O$, 3 × 20 ml de $NaHCO_3$ solution saturée puis 2 × 20 ml de $H_2O$. On sèche sur $Na_2SO_4$ sec. On évapore à sec. On obtient un produit blanc. (4,68 g), Rdt 89 %, Rf = 0,55 ($C_3$).

N-Boc-L. Tyrosyl-(Otbu) D. Sérine II

2,19 g (5 mM) de Boc Tyr-D Ser (Otbu) méthyl ester sont dissous dans 10 ml de méthanol et agités à 0 °C durant 3 h avec 10 ml de NaOH 2N. On évapore MeOH, on ajoute 10 ml d'eau, filtre la DCU et acidifie par HCl (pH 2). On extrait avec AcOEt (3 × 30 ml). On sèche le solvant organique et évapore à sec. On obtient un solide blanc. Rf = 0,52 ($C_2$).

Boc-Glycyl-L. Phénylalanine méthyl ester III

On dissout à 0 °C 5,25 g de Boc glycine dans 25 ml de THF anhydre. On ajoute successivement 6,47 g de L. Phe méthyl ester dans 20 ml de $CHCl_3$ sec, puis 4,2 ml de $NEt_3$ dans 10 ml de $CHCl_3$. Après agitation 10 mn, on ajoute 4,6 g de HOBT dans 20 ml de THF puis 6,19 g de DCC dissous dans 50 ml de $CHCl_3$. On agite 1 h à 0 °C puis 12 h à température ordinaire. On traite comme pour l'obtention du composé I. On obtient 10 g de produit. Rf = 0,45 ($C_3$).

Boc-Glycyl-L. Phénylalanine IV

10 g du composé précédent sont dissous dans 60 ml de MeOH. On agite à 0° durant 1 h puis 3 h à température ordinaire. On traite comme pour l'obtention de Boc-Tyr-(Otbu) D. Ser.

On obtient un solide recristallisé dans AcOET (7,2 g).

Boc-Gly-L. Phe-L. Leu méthyl ester V

La réaction de condensation est effectuée comme pour le composé I à partir de 6,7 g de tboc Gly-Phe OH et 3,81 g de L. Leu méthyl ester (chlorhydrate). On obtient 7,64 g d'un composé recristallisé dans AcOEt. Rf = 0,7 ($C_3$).

Trifluoroacétate de Boc-Gly-L. Phe-L. Leu-$OCH_3$ VI

4,64 g du composé V sont mis en solution dans 15 ml de TFA. On laisse agiter 30 mn à 0 °C puis 30 mn à température ordinaire. On chasse TFA sous vide poussé (13,3 Pa). Le résidu sirupeux est agité avec 20 ml d'éther sec (10 fois successivement) et l'éther est séparé par filtration. Lorsque l'éther est neutre, la poudre blanche obtenue est essorée rapidement puis séchée sous vide poussé. On obtient 3,07 g de produit. Rf = 0,5 ($C_2$).

Boc-Tyr-(Otbu) D. Ser-Gly-Phe-Leu-$OCH_3$ VII

1,69 g du composé II sont dissous dans 15 ml de THF sec. On ajoute à 0 °C, une solution de 1,85 g de VI et 0,56 ml et $NEt_3$ dans 15 ml de $CHCl_3$. Après 10 mn, on ajoute successivement 0,61 g de HOBT dans 5 ml de THF puis 0,82 g de DCC dans 5 ml de $CHCl_3$. On agite à 0 °C 1 h, puis 20 h à température ordinaire. On traite comme dans l'exemple I. On obtient 1,75 g d'un solide blanc. Rdt = 60 %. Rf = 0,65 ($C_3$).

Boc-Tyr-(Otbu) D. Ser-Gly-Phe-Leu-OH VIII

1,73 g de VII sont agités à 0 °C dans 70 ml de MeOH et 4,6 ml de NaOH N. Après 1 h à 0 °C, on laisse 12 h à température ambiante. On obtient un solide (1,33 g). Rdt = 80 %. Rf = 0,45 ($C_2$).

Boc-Tyr-(Otbu) D. Ser-Gly-Phe-Leu-(Otbu)Thr-$OCH_3$ IX

Le couplage est effectué entre 1,33 g de VIII et 0,41 g de l'ester méthylique de la L. Threonine (HCl) dans des conditions similaires à l'obtention de I ou VII.

On obtient 1,5 g d'un solide blanc. Rdt = 90 %. Rf = 0,67 ($C_3$).

Boc-Tyr-(Otbu) D. Ser-Gly-Phe-Leu-(Otbu)Thr-OH X

La saponification est effectuée sur 1,5 g du composé IX et donne, selon la méthode fournissant le composé VIII, un produit solide (1,26 g). Rf = 0,8 ($C_1$).

H-Tyr-D. Ser-Gly-Phe-Leu-Thr-OH XI

0,96 g de X sont mis à agiter 30 mn à 0 °C dans 1 ml de TFA saturé d'HCl gazeux. On évapore à sec sous 13,3 Pa. On ajoute de l'éther sec et on triture 15 fois avec 10 ml.

On obtient un produit blanc crème (0,41 g). Le composé est purifié par passage sur une colonne de LH 20, en utilisant le MeOH comme solvant. Le produit blanc obtenu est lyophylisé. Sa pureté est contrôlée par HPLC. (Colonne microbondapak $C_{18}$) solvant : $ACO^{(-)}NH_4^{(+)}$. $10^{-2}$ M, pH = 4,2 (80 %) et $CH_3CN$ (20 %). Débit 1,2 ml mn -1. Temps rétention 10,2 mn.

Le tableau I suivant donne les formules des composés de formule générale I que l'on a préparés selon les procédés de l'exemple détaillé précédemment ou par des variantes des techniques classiquement employées dans la synthèse des peptides.

Tableau I

| N° | FORMULE | Rf |
|---|---|---|
| 1 | Tyr -D.Ser -Gly-Phe-Leu-Thr | 0,51 ($C_1$) |
| 2 | Tyr-D. Ser-Gly-Phe-Leu-Ser | 0,62 ($C_1$) |
| 3 | Tyr-D. Thr-Gly-Phe-Leu-Ser | 0,58 ($C_1$) |
| 4 | Tyr-Aib-Gly-Phe-Leu-Ser | 0,35 ($C_1$) |

Tableau I (suite)

| N° | FORMULE | Rf |
|---|---|---|
| 5 | Tyr-AzaGly-Gly-Phe-Leu-Ser | 0,44 ($C_1$) |
| 6 | Tyr-Aib-Gly-Phe-Leu-Thr | 0,57 ($C_1$) |
| 7 | Tyr-AzaGly-Gly-Phe-Leu-Thr | 0,40 ($C_1$) |
| 8 | Tyr-D. Ser-Gly-Phe-Leu-NH$(CH_2)_2$OH | 0,30 ($C_1$) |
| 9 | Tyr-D. Ser-Gly-Phe-Leu-NH$(CH_2)_3$OH | 0,30 ($C_1$) |
| 10 | Tyr-D. Ser-Gly-Phe-Leu-Thr $NH_2$ | 0,12 ($C_1$) |
| 11 | Tyr-D. Ser (Obenzyl)-Gly-Phe-Leu-Thr | 0,50 ($C_2$) |
| 12 | Tyr-D. Ser(Obenzyl)-Gly-Phe-Leu-Thr(Obenzyl) | 0,47 ($C_3$) |
| 13 | Tyr-D. Ser-Gly-Phe-Leu-Thr OMe | 0,30 ($C_2$) |
| 14 | Tyr-D. Ser (Obenzyl)-Gly-Phe-Leu-Thr OMe | 0,52 ($C_3$) |
| 15 | Tyr-D. Ser(Op.fluorobenzyl)-Gly-Phe-Leu-Thr. O hexa fluorobenzyl | 0,61 ($C_3$) |
| 16 | Tyr-D. Ser-Gly-p. F. Phe-Leu-Thr | 0,57 ($C_1$) |
| 17 | Tyr-AzaGly-Gly-Phe-p. F. -Leu-Thr | 0,46 ($C_1$) |
| 18 | Tyr-AzaGly-Gly-Phe-Leu-Thr-O hexafluoro-benzyle | 0,80 ($C_2$) |
| 19 | Tyr-D. Ser (OCO p. fluorophényl)-Gly-Phe-Leu-Thr-Obenzyle | 0,75 ($C_3$) |
| 20 | Tyr-D. Ser (OCO p. fluorophényl)-Gly-Phe-Leu-Thr (OCO p. fluorophényl)O-benzyle | 0,81 ($C_3$) |
| 21 | Tyr-Aib-Gly-Phe-Leu-Thr-O hexafluoro-benzyle | 0,50 ($C_2$) |
| 22 | Tyr-Aib-Gly-penta fluoro Phe-Leu-Thr-O hexafluorobenzyle | 0,55 ($C_2$) |
| 23 | Tyr-D. Ser (OCO p. fluorobenzyle)-Gly-p. F. Phe-Leu-Thr-O Me | 0,48 ($C_3$) |
| 24 | Tyr-D. Ser-Gly-Phe-N. Leu-Thr | 0,50 ($C_1$) |
| 25 | Tyr-D. Ser-Gly-Phe-Ile-Thr | 0,50 ($C_1$) |
| 26 | Tyr-D. Ser-Gly-Phe-Ile-Ser | 0,58 ($C_1$) |
| 27 | Tyr-D. Ser-(OCO p. fluorobenzyle)-Gly-Phe-Leu-Ser-O hexafluorobenzyle | 0,75 ($C_3$) |
| 28 | Tyr-D. Ser $(OCOCH_3)$-Gly-Phe-Leu-Ser $(OCOCH_3)$ O hexafluorobenzyle | 0,70 ($C_3$) |
| 29 | Tyr-D. Ser-Gly-Phe-Leu-NH $(CH_2)_2$-OCO hexafluorobenzyle | ND |
| 30 | Tyr-D. Ser$(OCOCH_3)$-Gly-Phe-Leu-Thr-O hexa-fluorobenzyle | ND |

Tableau I (suite)

| N° | FORMULE | Rf |
|---|---|---|
| 31 | Tyr-D. Cys-Gly-Phe-Leu-Thr | 0,25 ($C_1$) |
| 32 | Tyr-D. Cys ($SCOC_6H_5$)-Gly-Phe-Leu-Thr-Ohexafluorobenzyle | ND |
| 33 | Tyr-D. Ser-Gly-Phe-Leu-Thr-NH-$CH_2$-$C_6H_5$ | 0,40 ($C_1$) |
| 34 | Tyr-D. Ser-Gly-Phe-Leu-Thr-NH-$CH_2$-$C_6F_5$ | ND |
| 35 | Tyr-D.Ser (OCO p. fluorophényl) Gly-Phe-Leu-Thr-NH $CH_2$ $C_6$ $F_5$ | 0,62 ($C_3$) |
| 36 | Tyr-D. Ser-Gly-Phe-Leu-Thr-NH-phosphatidyléthanolamine | ND |
| 37 | Tyr-β Phe. Ser-Gly-Phe-Leu-Thr | ND |
| 38 | Tyr-β OH Leu-Gly-Phe-Leu-Thr | ND |
| 39 | Tyr-4 OH Ile-Gly-Phe-Leu-Thr | ND |
| 40 | Tyr-α OH Nor Val-Gly-Phe-Leu-Thr | ND |
| 41 | Tyr-β OH Nor Val-Gly-Phe-Leu-Thr | ND |
| 42 | Tyr-γ OH Nor Val-Gly-Phe-Leu-Thr. | ND |
| 43 | Tyr-D. Ser-Gly-Phe-Leu-Thr-NH$(CH_2)_2$N(→O)-$(CH_3)_2$ | 0,50 ($C_2$) |
| 44 | Tyr-D.Ser-Gly-Phe.D.Leu | |
| 45 | Tyr-D.Thr-Gly-Phe.D.Leu | |
| 46 | Tyr-D.Ser-Gly-pFPhe-D.Leu-O-benzyle | |
| 47 | Tyr-D.Ser-Gly-pFPhe-D.Leu-O-benzyle | |

Dans ce tableau, les groupes entre parenthèses correspondent à des substituants fixés sur les chaînes latérales.

Comme indiqué précédemment, les composés de formule I présentent des propriétés pharmacologiques particulièrement intéressantes, à savoir principalement une action préférentielle sur les récepteurs δ des opiacés et une action analgésique. Leur toxicité n'apparaît qu'à des doses très supérieures aux doses pharmacologiquement actives, ce qui permet leur utilisation en thérapeutique, notamment comme analgésiques, psychotropes et antidiarrhéiques.

On donnera ci-après des résultats des études pharmacologiques et toxicologiques qui mettent en évidence ces propriétés.

1) Action in vitro sur les récepteurs μ et δ

L'étude de l'action soit sur les récepteurs μ soit sur les récepteurs δ a été faite en fonction de l'effet sur l'iléon isolé de cobaye ou sur le vas deferens de souris.

L'action sur les récepteurs μ a été déterminée par mesure de la concentration inhibitrice 50 % (IC 50) de la contraction de l'iléon isolé de cobaye, induite par stimulation coaxiale (PATON W.D.M., Brit. J. Pharm. 1959, *12*, 119).

L'action sur les récepteurs δ a été déterminée par mesure de la concentration inhibitrice 50 % (IC 50) de la contraction du vas deferens de souris induite par stimulation électrique.

Les concentrations inhibitrices 50 sont exprimées en concentration nanomolaire/litre. Chaque produit est testé par rapport à la Met-Enképhaline comme substance de référence et le résultat est exprimé également en % d'activité par rapport à la Met-Enképhaline = 100.

Le rapport R = (IC 50 iléon)/(IC 50 vas deferens) permet de voir si le produit étudié est μ ou δ spécifique. Les résultats sont donnés dans le tableau suivant. Dans ce tableau, le nombre d'essais est indiqué entre parenthèses.

Tableau II

| PRODUIT | ILEON | | VAS DEFERENS | | R |
|---|---|---|---|---|---|
| | IC 50 (nM) | Act. relative | IC 50 (nM) | Act. relative | IC 50 iléon / IC 50 V.D |
| Met-Enképhaline | 354 (6) | 100 | 18 (4) | 100 | 19,6 |
| Exemple 1 | 637 (5) | 55 | 0,80 (5) | 2250 | 796 |
| Exemple 24 | 1050 (2) | 34 | 1,2 (2) | 1500 | 875 |
| Exemple 44 | 247 (4) | 143 | 1,15 (4) | 1565 | 215 |
| Exemple 13 du brevet FR 78 12 543 (*) | 320 | 110 | 650 | 2,7 | 0,49 |

(*) Composé de formule

Tyr-D.Ala-Gly-Phe-Met-NH-$(CH_2)_5$-$SO_2$ NH

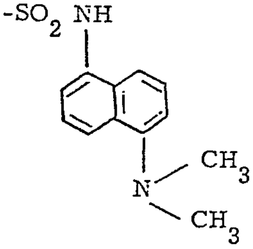

Ce tableau met en évidence l'effet δ spécifique des composés de formule I.

2) Activité analgésique

Les composés ont été testés « in vivo » par voie intraveineuse (i. v.) dans l'épreuve de la plaque chaude à 65 °C chez la souris, selon la méthode de Jacob et Blozovski (Arch. Int. Pharmacodyn. 1961, *33,* 296) par mesure du réflexe de lèchement de 15 minutes, de bond à 30 minutes et de saut ajusté à 60 et à 90 minutes.

La substance de référence était la $DAla_2$Met-Enképhaline.

Les résultats sont donnés au Tableau III.

Tableau III

| Traitement | N | Temps de réaction (s) | | | |
|---|---|---|---|---|---|
| | | 15' Lèchement | 30' Bond | 60' Saut ajusté | 90' Saut ajusté |
| Témoins i.v. | 6 | 4,5 ± 0,8 | 2,9 ± 2,2 | 13,4 ± 6,6 | 13,4 ± 5,3 |
| D.$Ala_2$Met-Enképhaline 100 mg/kg i.v. | 6 | 5,5 ± 1 | 5,5 ± 1,6 | 5,5 ± 1,2 | 5,6 ± 2 |

Tableau III (suite)

| Traitement | N | Temps de réaction (s) | | | |
|---|---|---|---|---|---|
| | | 15' Lèchement | 30' Bond | 60' Saut ajusté | 90' Saut ajusté |
| Ex. 1 25 mg/kg i.v. | 6 | 29,4 ± 3,2 | 30,1 ± 5,7 | 28,6 ± 7,2 | 32,2 ± 9,2 |
| Ex. 24 75 mg/kg i.v. | 6 | 37,4$^{x}$± 1,6 | 34,6$^{x}$± 6,3 | 35,4$^{x}$± 8 | 30,4$^{x}$± 9 |

$^{x}p < 0,05$

3) Test de susceptibilité à la naloxone — Détermination de l'apparition de la dépendance

Ce test permet d'évaluer la dépendance provoquée par l'administration chronique d'analgésiques morphiniques. Il consiste à provoquer la crise de dépendance après 10 jours de traitement par injection d'une dose élevée d'un antagoniste : la naloxone. Le composé 1 a été testé par administration ICV chez le rat selon la technique de Frederickson et al., Res. Common, Pathol. Pharmacol. *5*, 867 (1973).

Les résultats sont rapportés dans le tableau IV et comparés à ceux obtenus avec la morphine.

Tableau IV

| Traitement | N | Naloxone | Test d'abstinence (sauts) |
|---|---|---|---|
| Témoins ICV (saline) | 5 | 10 mg/kg S.C. au 10ème jour | 12 ± 3 |
| Composé 1 25 mg/kg 4fois par jour durant 10 jours | 5 5 | id. | 10 ± 2 |
| Morphine 25 mg/kg 4 fois par jour durant 10 jours | 5 | id. | 74 ± 11 |

4) Action antidiarrhéique

Elle a été recherchée par l'épreuve à l'huile de ricin chez le rat selon la méthode de Niemegeers et coll. (Arzn.-Forsch. *22*, 516-518, 1972).

Ces essais ont été réalisés avec des rats mâles de 160 à 220 g. Après un jeûne de 18 heures, chaque animal a reçu par voie orale, un millilitre d'huile de ricin.

Ces composés en solution dans l'eau ont été administrés par voie orale sous un volume de 0,5 ml pour 100 grammes de poids corporel, une heure avant l'huile de ricin.

Les résultats sont donnés dans le Tableau V.

Tableau V

| Séries | Nombre de rats | Nombre d'animaux non diarrhéiques | |
|---|---|---|---|
| | | 2 heures | 4 heures |
| Témoin x | 30 | 8 | 2 |
| Exemple 24<br>3 mg/kg | 10 | 10 | 6 |
| Codéine base<br>3 mg/kg<br>10 mg/kg | <br>10<br>10 | <br>7<br>10 | <br>4<br>7 |
| Morphine (chlorhydrate)<br>1 mg/kg<br>3 mg/kg | <br>10<br>10 | <br>9<br>10 | <br>4<br>6 |

(*) Huile de ricin seule.

5) Action des composés δ spécifiques revendiqués sur le métabolisme de la dopamine qui est caractéristique de l'activité psychotrope considérée

L'action sélective du composé 1 sur la libération de dopamine est démontrée par des expériences d'injection ICV de morphine, de $DAla_2$ Met. Eukamide et du composé 1. (Chenelet et al., Nature (1981) *291*, 320.

La libération spontanée de dopamine est augmentée de 50 % par la morphine ($10^{-6}$ M), et de 100 % par la $DAla_2$ Met. Eukamide ($10^{-6}$ M) et de 100 % également par le composé 1 à une dose ($5 \times 10^{-8}$ M) 20 fois moindre que le peptide précédent qui ne manifeste qu'une faible préférence pour les récepteurs δ.

Cet effet préférentiel du composé δ-spécifique 1 sur les récepteurs dopaminergiques peut être mis en évidence par administration i. v. du composé 1 et mesure de l'activité locomotrice selon Stinus et al. Proc. Natl. Acad. Sci. USA *77*, 2323. (1980). Les résultats sont rapportés dans le tableau VI. Chaque expérience a été réalisée sur un lot de 10 animaux (rats mâles 200-250 g) et les résultats comparés avec un lot témoin.

Tableau VI

| Composés | Activité locomotrice |
|---|---|
| Solution saline physiologique | 100 ± 15 % |
| Composé 1<br>(5 mg/kg) | 65 ± 10 % |

6) Toxicité aiguë

La détermination de la mortalité chez la souris est réalisée à la suite de l'administration unique par voie intra-veineuse de doses croissantes des composés à tester.

La $DL_{50}$ pour tous les composés de formule I étudiés est supérieure à 300 mg/kg i. v.

Le médicament de l'invention est administrable à l'homme par la voie orale sous forme de comprimés, comprimés dragéifiés, capsules, gouttes ou sirops. Il peut aussi être présenté, pour l'administration parentérale sous forme de soluté injectable.

Chaque dose unitaire contient avantageusement de 0,2 à 100 mg de principe actif, ainsi

— pour les comprimés de 0,2 à 30 mg de principe actif

— pour les suppositoires de 10 à 50 mg de principe actif

— pour les ampoules injectables de 1 ml, une solution de 0,5 à 2 % de principe actif dans une solution stérile physiologique.

Les doses administrables journellement peuvent varier de 5 mg à 200 mg de principe actif environ.

Le médicament de l'invention peut être administré à l'homme pour le traitement de la douleur ou des diarrhées.

En raison de son activité psychotrope, il est également applicable au traitement des psychoses et névroses.

A titre d'exemple les comprimés contenant 10 mg de principe actif répondent à la formule suivante

| | |
|---|---|
| Composé 1 | 10 mg |
| Amidon de maïs | 100 mg |
| Lactose | 380 mg |
| Gélatine | 4 mg |
| Stéarate de magnésium | 6 mg |
| Comprimé terminé à | 500 mg |

**Revendications** (pour les Etats contractants : BE, CH, DE, FR, GB, IT, LI, LU, NL, SE)

1. Dérivés de peptides de formule

Tyr-A-Gly-B-C-D (I)

dans laquelle

A est un reste AzaGly, Aib, D. Ser, D. Thr, D. Cys, homo Sérine, β Phe Ser, β OH Leu, 4OH Ile, α, β, γ OHNor Val ou OH Val dont les groupes OH ou SH latéraux, lorsqu'ils existent, peuvent être libres ou protégés

i) par un radical alkyle simple ou ramifié ayant de 1 à 6 atomes de carbone,

ii) par un radical phényle non substitué ou substitué par un ou plusieurs atomes de fluor,

iii) par un radical benzyle non substitué ou substitué par un ou plusieurs atomes de fluor,

iv) par un radical acyle aliphatique ayant de 1 à 6 atomes de carbone ou par un radical acyle COX dans lesquels X est un radical phényle, benzyle ou benzyhydryle, éventuellement substitué par un ou plusieurs atomes de fluor,

B est un reste L. Phe, pF. L. Phe ou pentafluoro L. Phe,

C est un reste Leu, N. Leu ou Ile,

D représente un groupe

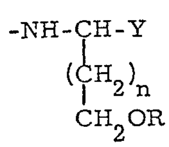

ou

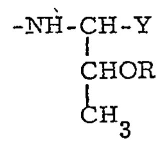

dans lequel

n = 0, 1 ou 2,

R est un atome d'hydrogène, ou un radical tel que défini pour la protection du groupe OH du reste A,

Y est un atome d'hydrogène, un groupe hydroxy, carboxy, carbamoyle, un groupe $OR_1$, $COOR_1$ ou $CONHR_1$ dans lequel $R_1$ représente un radical tel que défini pour la protection du groupe OH du reste A, une chaîne phosphatidyléthanolamine ou une chaîne

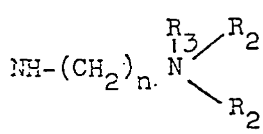

dans laquelle n est un nombre entier de 0 à 3, $R_2$ est un atome d'hydrogène, ou un radical alkyle simple ayant de 1 à 4 atomes de carbone et $R_3$ est un atome d'hydrogène ou d'oxygène ou un reste alkyle simple ayant de 1 à 4 atomes de carbone et leurs sels pharmaceutiquement acceptables.

2. Peptide de formule Tyr-D. Ser-Gly-Phe-N-Leu-Thr et ses sels pharmaceutiquement acceptables.

3. Peptide de formule Tyr-D. Ser-Gly-Phe-Leu-Thr et ses sels pharmaceutiquement acceptables.

4. Composition thérapeutique, caractérisée en ce qu'elle contient à titre de principe actif un composé selon l'une quelconque des revendications 1 à 3.

**Revendications** (pour l'Etat contractant AT)

1. Procédé de préparation de dérivés de peptides de formule

Tyr-A-Gly-B-C-D (I)

dans laquelle

A est un reste AzaGly, Aib, D. Ser, D. Thr, D. Cys, homo Sérine, β Phe Ser, β OH Leu, 4OH Ile, α, β, γ OHNor Val ou OH Val dont les groupes OH ou SH latéraux, lorsqu'ils existent, peuvent être libres ou protégés ;

i) par un radical alkyle simple ou ramifié ayant de 1 à 6 atomes de carbone,

ii) par un radical phényle non substitué ou substitué par un ou plusieurs atomes de fluor,

iii) par un radical benzyle non substitué ou substitué par un ou plusieurs atomes de fluor,

iv) par un radical acyle aliphatique ayant de 1 à 6 atomes de carbone ou par un radical acyle COX dans lesquels X est un radical phényle, benzyle ou benzyhydryle, éventuellement substitué par un ou plusieurs atomes de fluor,

B est un reste L. Phe, pF. L. Phe ou pentafluoro L. Phe,

C est un reste Leu, N. Leu ou Ile,

D représente un groupe

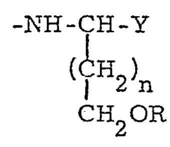

ou

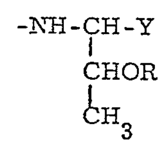

dans lequel

n = 0, 1 ou 2,

R est un atome d'hydrogène ou un radical tel que défini pour la protection du groupe OH du reste A,

Y est un atome d'hydrogène, un groupe hydroxy, carboxy, carbamoyle, un groupe $OR_1$, $COOR_1$ ou $CONHR_1$ dans lequel $R_1$ représente un radical tel que défini pour la protection du groupe OH du reste A, une chaîne phosphatidyléthanolamine ou une chaîne

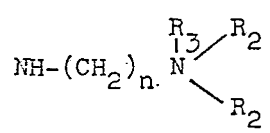

dans laquelle n est un nombre entier de 0 à 3, $R_2$ est un atome d'hydrogène, ou un radical alkyle simple ayant de 1 à 4 atomes de carbone et $R_3$ est un atome d'hydrogène ou d'oxygène ou un reste alkyle simple ayant de 1 à 4 atomes de carbone et leurs sels pharmaceutiquement acceptables, caractérisé en ce qu'on utilise les procédés classiques de la synthèse peptidique.

2. Procédé de préparation de peptide de formule Tyr-D. Ser-Gly-Phe-N Leu-Thr et ses sels pharmaceutiquement acceptables, caractérisé en ce qu'on utilise les procédés classiques de la synthèse peptidique.

3. Procédé de préparation de formule Tyr-D. Ser-Gly-Phe-Leu-Thr et ses sels pharmaceutiquement acceptables caractérisé en ce qu'on utilise les procédés classiques de la synthèse peptidique.

4. Procédé de préparation d'une composition thérapeutique, caractérisé en ce que l'on met un composé de formule

Tyr-A-Gly-B-C-C (II)

dans laquelle

A est un reste AzaGly, Aib, D. Ser, D. Thr, D. Cys, homo Sérine, β Phe Ser, β OH Leu, 4OH Ile, α, β, γ OHNor Val ou OH Val dont les groupes OH ou SH latéraux, lorsqu'ils existent, peuvent être libres ou protégés

i) par un radical alkyle simple ou ramifié ayant de 1 à 6 atomes de carbone ;

ii) par un radical phényle non substitué ou substitué par un ou plusieurs atomes de fluor ;

iii) par un radical benzyle non substitué ou substitué par un ou plusieurs atomes de fluor ;

iv) par un radical acyle aliphatique ayant de 1 à 6 atomes de carbone ou par un radical acyle COX dans lesquels X est un radical phényle, benzyle ou benzyhydryle, éventuellement substitué par un ou plusieurs atomes de fluor.

B est un reste L. Phe, pF. L. Phe ou pentafluoro L. Phe,

C est un reste Leu, N. Leu ou Ile,

D représente un groupe

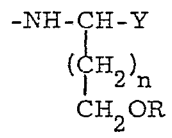

ou

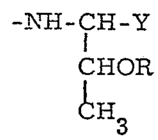

dans lequel

n = 0, 1 ou 2,

R est un atome d'hydrogène ou un radical tel que défini pour la protection du groupe OH du reste A,

Y est un atome d'hydrogène, un groupe hydroxy, carboxy, carbamoyle, un groupe $OR_1$, $COOR_1$ ou $CONHR_1$ dans lequel $R_1$ représente un radical tel que défini pour la protection du groupe OH du reste A, une chaîne phosphatidyléthanolamine ou une chaîne

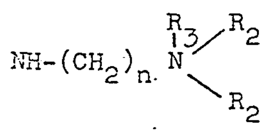

dans laquelle n est un nombre entier de 0 à 3, $R_2$ est un atome d'hydrogène, ou un radical alkyle simple ayant de 1 à 4 atomes de carbone et $R_3$ est un atome d'hydrogène ou d'oxygène ou un reste alkyle simple ayant de 1 à 4 atomes de carbone ou l'un de ses sels pharmaceutiquement acceptable sous une forme pharmaceutique.

**Claims** (for the Contracting States : BE, CH, DE, FR, GB, IT, LI, LU, NL, SE)

1 Peptide derivatives having the formula

Tyr-A-Gly-B-C-D (I)

in which

A is a AzaGly, Aib, D. Ser, D. Thr, D. Cys, homo Serine, β Phe Ser, β OH Leu, 4OH Ile, α, β, γ OHNor-Val or OH Val residue in which the side-chain OH or SH groups, when same exist, may be free or protected by

i) a straight- or branched-chain alkyl radical having 1-6 carbon atoms,

ii) an unsubstituted phenyl radical or a phenyl radical substituted with one or more fluorine atoms,

iii) an unsubstituted benzyl radical or a benzyl radical substituted with one or more fluorine atoms,

iv) an aliphatic acyl radical having 1-6 carbon atoms or an acyl radical of the formula COX in which X is a phenyl, benzyl or benzhydril radical, optionally susbtituted with one or more fluorine atoms,

B, is a L. Phe, pF. L. Phe or pentafluoro L. Phe residue,

C is a Leu, N. Leu or Ile residue,

D represents a group

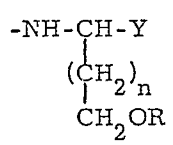

or

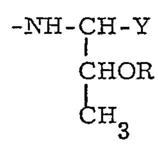

in which

n = 0, 1 or 2,

R is a hydrogen atom or a radical as defined for the protection of the OH group of residue A,

Y is hydrogen atom, a hydroxy, carboxy, carbamoyl group, a group $OR_1$, $COOR_1$ or $CONHR_1$ in which $R_1$ represents a radical such as defined for the protection of the OH group of residue A, a phosphatidylethanolamine chain, or a chain

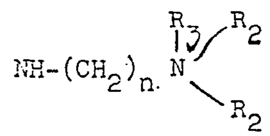

in which n is an integer from 0 to 3, $R_2$ is a hydrogen atom or a straight alkyl radical containing 1-4 carbon atoms, and $R_3$ is a hydrogen or oxygen atom or a straight alkyl residue having 1-4 carbon atoms and their pharmaceutically acceptable salts.

2. Peptide having the formula Tyr-D. Ser-Gly-Phe-N. Leu-Thr and its pharmaceutically acceptable salts.

3. Peptide having the formula Tyr-D. Ser-Gly-Phe-Leu-Thr and its pharmaceutically acceptable salts.

4. Therapeutic composition comprising, as active ingredient, a compound according to any one of claims 1 to 3.

**Claims** (for the Contracting State AT)

1. Process for preparing peptide derivatives having the formula

Tyr-A-Gly-B-C-D (I)

in which

A is a AzaGly, Aib, D. Ser, D. Thr, D. Cys, homo Serine, β Phe Ser, β OH Leu, 4OH, Ile, α, β, γ OHNor-Val or OH Val residue in which the side-chain OH or SH groups, when same exist, may be free or protected by

i) a straight- or branched-chain alkyl radical having 1-6 carbon atoms,

ii) an unsubstituted phenyl radical or a phenyl radical substituted with one or more fluorine atoms,

iii) an unsubstituted benzyl radical or a benzyl radical substituted with one or more fluorine atoms,

iv) an aliphatic acyl radical having 1-6 carbon atoms or an acyl radical of the formula COX in which X is a phenyl, benzyl or benzhydryl radical, optionally substituted with one or more fluorine atoms,

B is a L. Phe, pF. L. Phe or pentafluoro L. Phe residue,

C is a Leu, N. Leu or Ile residue,

D represents a group

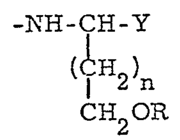

or

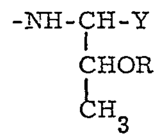

in which

n = 0, 1 or 2,

R is a hydrogen atom or a radical as defined for the protection of the OH group of residue A,

Y is a hydrogen atom, a hydroxy, carboxy, carbamoyl group, a group $OR_1$, $COOR_1$ or $CONHR_1$ in which $R_1$ represents a radical such as defined for the protection of the OH group of residue A, a phosphatidylethanolamine chain, or a chain

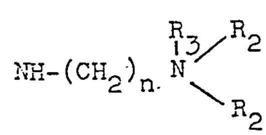

in which n is an integer from 0 to 3, $R_2$ is a hydrogen atom or a straight alkyl radical containing 1-4 carbon atoms, and $R_3$ is a hydrogen or oxygen atom or a straight alkyl residue having 1-4 carbon atoms and their pharmaceutically acceptable salts, characterized by the use of the classical processes of peptide synthesis.

2. Process for preparing a peptide having the formula Tyr-D. Ser-Gly-Phe-N. Leu-Thr and its pharmaceutically acceptable salts, characterized by the use of classical processes of peptide synthesis.

3. Process for preparing a peptide having the formula Tyr-D. Ser-Gly-Phe-Leu-Thr and its pharmaceutically acceptable salts, characterized by the use of classical processes of peptide synthesis.

4. Process for preparing a therapeutic composition, characterized by putting a compound having the formula

Tyr-A-Gly-B-C-D (I)

in which

A is a AzaGly, Aib, D. Ser, D. Thr, D. Cys, homo Serine, β, Phe Ser, β OH Leu, 4OH Ile, α, β, γ OHNor-Val or OH Val residue in which the side-chain OH or SH groups, when same exist, may be free or protected by

(i) a straight- or branched-chain alkyl radical having 1-6 carbon atoms,

(ii) an unsubstituted phenyl radical or a phenyl radical substituted with one or more fluorine atoms,

(iii) an unsubstituted benzyl radical or a benzyl radical substituted with one or more fluorine atoms,

(iv) an aliphatic acyl radical having 1-6 carbon atoms or an acyl radical of the formula COX in wich X is a phenyl, benzyl or benzhydryl radical, optionally substituted with one or more fluorine atoms,

B is a L. Phe, pF. L. Phe or pentafluoro L. Phe residue,

C is a Leu, N. Leu or Ile residue,

D represents a group

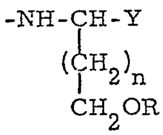

or

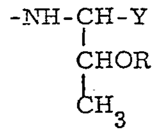

in which

n = 0, 1 or 2,

R is a hydrogen atom or a radical as defined for the protection of the OH group of residue A,

Y is a hydrogen atom, a hydroxy, carboxy, carbamoyl group, a group $OR_1$, $COOR_1$ or $CONHR_1$ in which $R_1$ represents a radical such as defined for the protection of the OH group of residue A, a phosphatidylethanolamine chain, or a chain

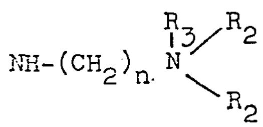

in which n is an integer from 0 to 3, $R_2$ is a hydrogen atom or a straight alkyl radical containing 1-4 carbon atoms, and $R_3$ is a hydrogen or oxygen atom or a straight alkyl residue having 1-4 carbon atoms and their pharmaceutically acceptable salts, in a pharmaceutical form.

**Ansprüche** (für die Vertragstaaten : BE, CH, DE, FR, GB, IT, LI, LU, NL, SE)

1. Peptidderivate der Formel

Tyr-A-Gly-B-C-D (I)

in der

A ein Rest der Formel AzaGly, Aib, D. Ser., D. Thr, D. Cys, homo Serin, β Phe Ser, β OH Leu, 4OH Ile, α, β, γ OHNor Val oder OH Val ist, deren OH- oder SH-Seitengruppen, wenn sie vorliegen, frei oder

i) durch einen einfachen oder verzweigten Alkylrest mit 1 bis 6 Kohlenstoffatomen,

ii) durch einen unsubstituierten oder durch ein oder mehrere Fluoratome substituierten Phenylrest,

iii) durch einen unsubstituierten oder durch ein oder mehrere Fluoratome substituierten Benzylrest,

iv) durch einen aliphatischen Acylrest mit 1 bis 6 C-Atomen oder durch einen Acylrest COX, in denen X ein gegebenenfalls mit einem oder mehreren Fluoratomen substituierter Phenyl-, Benzyl- oder Benzyhydrylrest ist, geschützt sein können,

B ein Rest L. Phe, pF. L. Phe oder Pentafluoro-L. Phe,

C ein Rest Leu, N. Leu oder Ile ist,

D eine Gruppe

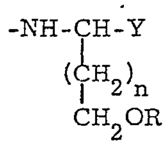

oder

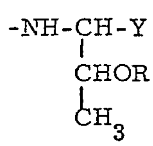

in der

n = 0, 1 oder 2, darstellt,

R ein Wasserstoffatom oder ein Rest, wie er für den Schutz der OH-Gruppe des Restes A definiert wurde, ist,

Y ein Wasserstoffatom, eine Hydroxyl-, Carboxyl-, Carbamoylgruppe, eine Gruppe $OR_1$, $COOR_1$ oder $CONHR_1$ ist, in der $R_1$ einen Rest, wie er für den Schutz der OH-Gruppe des Restes A definiert wurde, eine Phosphatidylethanolaminkette oder eine Kette

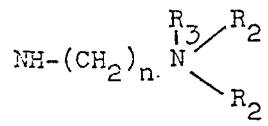

ist, in der n eine ganze Zahl von 0 bis 3, $R_2$ ein Wasserstoffatom oder ein einfacher Alkylrest mit 1 bis 4 Kohlenstoffatomen und $R_3$ ein Wasserstoff- oder Sauerstoffatom oder ein einfacher Alkylrest mit 1 bis 4 C-Atomen ist, und ihre pharmazeutisch annehmbaren Salze.

2. Peptid der Formel Tyr-D. Ser-Gly-Phe-N Leu-Thr und seine pharmazeutisch annehmbaren Salze.

3. Peptid der Formel Tyr-D. Ser-Gly-Phe-Leu-Thr und seine pharmazeutisch annehmbaren Salze.

4. Therapeutische Zusammensetzung, dadurch gekennzeichnet, daß sie als Wirkstoff eine Verbindung nach irgendeinem der Ansprüche 1 bis 3 enthält.

**Ansprüche** (für der Vertragsstaat AT)

1. Verfahren zur Herstellung von Peptidderivaten der Formel

Tyr-A-Gly-B-C-D (I)

in der

A ein Rest der Formel AzaGly, Aib, D. Ser., D. Thr, D. Cys, homo Serin, β Phe Ser, β OH Leu, 4OH Ile, α, β, γ OHNor Val oder OH Val ist, deren OH- oder SH-Seitengruppen, wenn sie vorliegen, frei oder

i) durch einen einfachen oder verzweigten Alkylrest mit 1 bis 6 Kohlenstoffatomen,

ii) durch einen unsubstituierten oder durch ein oder mehrere Fluoratome substituierten Phenylrest,

iii) durch einen unsubstituierten oder durch ein oder mehrere Fluoratome substituierten Benzylrest,

iv) durch einem aliphatischen Acylrest mit 1 bis 6 C-Atomen oder durch einen Acylrest COX, in denen X ein gegebenenfalls mit einem oder mehreren Fluoratomen substituierter Phenyl-, Benzyl- oder Benzyhydrylrest ist, geschützt sein können,

B ein Rest L. Phe, pF. L. Phe oder Pentafluoro-L. Phe,

C ein Rest Leu, N. Leu oder Ile ist,

D eine Gruppe

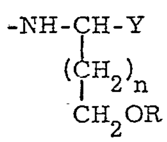

oder

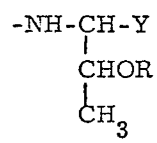

in der

n = 0, 1 oder 2, darstellt,

R ein Wasserstoffatom oder ein Rest, wie er für den Schutz der OH-Gruppe des Restes A definiert wurde, ist,

Y ein Wasserstoffatom, eine Hydroxyl-, Carboxyl-, Carbamoylgruppe, eine Gruppe $OR_1$, $COOR_1$ oder $CONHR_1$ ist, in der $R_1$ einen Rest, wie er für den Schutz der OH-Gruppe des Restes A definiert wurde, eine Phosphatidylethanolaminkette oder eine Kette

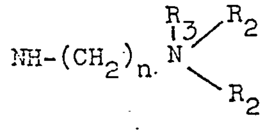

ist, in der n eine ganze Zahl von 0 bis 3, $R_2$ ein Wasserstoffatom oder ein einfacher Alkylrest mit 1 bis 4 Kohlenstoffatomen und $R_3$ ein Wasserstoff- oder Sauerstoffatom oder ein einfacher Alkylrest mit 1 bis 4 C-Atomen ist, und ihren pharmazeutisch annehmbaren Salzen, dadurch gekennzeichnet, daß man die klassischen Verfahren der Peptidsynthese anwendet.

2. Verfahren zur Herstellung eines Peptids der Formel Tyr-D. Ser-Gly-Phe-N Leu-Thr und seiner

pharmazeutisch annehmbaren Salze, dadurch gekennzeichnet, daß man die klassischen Verfahren der Peptidsynthese anwendet.

3. Verfahren zur Herstellung der Formel Tyr-D. Ser-Gly-Phe-Leu-Thr und seiner pharmazeutisch annehmbaren Salze, dadurch gekennzeichnet, daß man die klassischen Verfahren der Peptidsynthese anwendet.

4. Verfahren zur Herstellung einer therapeutischen Zusammensetzung, dadurch gekennzeichnet, daß man eine Verbindung der Formel

Tyr-A-Gly-B-C-D (I)

in der

A ein Rest der Formel AzaGly, Aib, D. Ser., D. Thr, D. Cys, homo Serin, β Phe Ser, β OH Leu, 4OH Ile, α, β, γ OHNor Val oder OH Val ist, deren OH- oder SH-Seitengruppen, wenn sie vorliegen frei oder

i) durch einen einfachen oder verzweigten Alkylrest mit 1 bis 6 Kohlenstoffatomen,

ii) durch einen unsubstituierten oder durch ein oder mehrere Fluoratome substituierten Phenylrest,

iii) durch einen unsubstituierten oder durch ein oder mehrere Fluoratome substituierten Benzylrest,

iv) durch einen aliphatischen Acylrest mit 1 bis 6 C-Atomen oder durch einen Acylrest COX, in denen X ein gegebenenfalls mit einem oder mehreren Fluoratomen substituierter Phenyl-, Benzyl- oder Benzyhydrylrest ist, geschützt sein können,

B ein Rest L. Phe, pF. L. Phe oder Pentafluoro-L. Phe,

C ein Rest Leu, N. Leu oder Ile ist,

D eine Gruppe

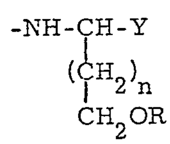

oder

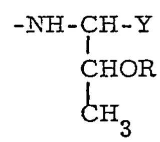

,

in der

n = 0, 1 oder 2, darstellt,

R ein Wasserstoffatom oder ein Rest, wie er für den Schutz der OH-Gruppe des Restes A definiert wurde, ist,

Y ein Wasserstoffatom, eine Hydroxyl- Carboxyl-, Carbamoylgruppe, eine Gruppe $OR_1$, $COOR_1$ oder $CONHR_1$ ist, in der $R_1$ einen Rest, wie er für den Schutz der OH-Gruppe des Restes A definiert wurde, eine Phosphatidylethanolaminkette oder eine Kette

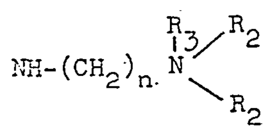

ist, in der n eine ganze Zahl von 0 bis 3, $R_2$ ein Wasserstoffatom oder ein einfacher Alkylrest mit 1 bis 4 Kohlenstoffatomen und $R_3$ ein Wasserstoff- oder Sauerstoffatom oder ein einfacher Alkylrest mit 1 bis 4 C-Atomen ist, oder eines ihrer pharmazeutisch annehmbaren Salze in eine pharmazeutische Form bringt.